# EUROPÄISCHE PATENTANMELDUNG

(11) **EP 0 938 882 A2**
(43) Veröffentlichungstag der Anmeldung: **01.09.1999**
(21) Anmeldenummer: 99102437.3
(22) Anmeldetag: 09.02.1999
(51) Int. Cl.: A61F 13/02, A61F 15/00

(54) **Trennpapier mit mehreren auf diesem befindlichen, im wesentlichen parallel angeordneten Pflaster**

(30) Priorität: 25.02.1998 DE 19807970
(71) Anmelder: Beiersdorf Aktiengesellschaft, 20245 Hamburg (DE)
(72) Erfinder: Schultz, Günther, 22457 Hamburg (DE)

(57) **Zusammenfassung**

Trennpapier 1 mit mehreren auf dieser befindlichen, im wesentlichen parallel nebeneinander angeordneten Pflastern 2, 3, 4, 5, 6, die jeweils aus einem Trägermaterial 21 bestehen, das einseitig mit einer selbstklebenden Beschichtung und gegebenenfalls mit einem Wundabdeckmaterial 22 versehen ist, wobei das Trennpapier 1 zwischen jedem der Pflaster 2, 3, 4, 5, 6 eine Längsperforation 11 aufweist, wobei das Trennpapier 1 eine Querperforation 12 aufweist, die unterhalb der Pflaster 2, 3, 4, 5, 6 verläuft und wobei die Längsperforation 11 ausgehend von einer Kante des Trennpapiers 1 zumindest bis zur Querperforation 12 ausgebildet ist.

## Beschreibung

Die Erfindung betrifft ein Trennpapier mit mehreren auf diesem befindlichen, im wesentlichen parallel angeordneten Pflaster, die aus einem Trägermaterial bestehen, das einseitig mit einer selbstklebenden Beschichtung und gegebenenfalls mit einem Wundabdeckmaterial versehen ist.

Die DE P 41 21 189 beschreibt verpackte Pflaster, insbesondere Wundpflaster mit einer Wundabdeckung oder Verbandmaterialien oder Klebematerialien für medizinische Zwecke mit jeweils einem ebenen Träger, wobei der Träger mit einer Haftkleberschicht und gegebenenfalls einer Wundabdeckung versehen ist, die mit einem Abdeckstreifen bedeckt und geschützt sind, wobei der Abdeckstreifen an dieser Haftkleberschicht haftet, und wobei diese Pflaster oder Materialien von zwei Verpackungsblättern, die eine Verpackung bilden, umhüllt und eingeschlossen sind.

Ein Verpackungsblatt liegt über der Außenseite des genannten Abdeckstreifens und ist auf seiner Innenseite mit einer Klebemasse oder Siegelmasse versehen und mit der Außenseite des Abdeckstreifens verklebt oder versiegelt, wodurch der Abdeckstreifen fester an dem Verpackungsblatt als an der Haftkleberschicht haftet, und wobei die beiden Verpackungsblätter in einem über die Fläche des Abdeckstreifens hinausgehenden Bereich miteinander verklebt oder versiegelt sind und sich die beiden Verpackungsblätter voneinander trennen lassen.

Das nicht mit der Klebmasse oder Siegelmasse versehene Verpackungsblatt kann gegebenenfalls auch vollständig entfernt werden, und die genannten Pflaster oder Materialien hatten mit dem Abdeckstreifen auf dem anderen, mit der Klebmasse oder Siegelmasse versehenen Verpackungsblatt in der Weise, daß die Haftkleberschicht und gegebenenfalls die Wundabdeckung bedeckt und geschützt sind.

Aus der DE 19 36 606 C3 ist ein Wundschnellverband bekannt, der aus einer Mehrzahl durch Perforationslinien leicht voneinander trennbarer Einzelpflaster aus jeweils einem mit einer Haftkleberschicht vorgesehenen Trägerstreifen, einer darauf aufgebrachten Wundauflage und einer darüber angeordneten Schutzabdeckung besteht. Die Schutzabdeckung jedes Einzelpflasters ist als flächiges Einstück ausgebildet, und an die Schutzabdeckung angrenzend ist eine als umgelegter Streifen ausgebildete von der Schutzabdeckung zum Teil überlappte Fahne angeordnet, deren einer umgelegter Teil mit der Haftkleberschicht verbunden ist.

Dann offenbart die GB 549,736 in einer Reihe angeordnete Pflaster mit einer Klebebeschichtung und einer Wundauflage, die über Perforationslinien miteinander verknüpft sind.

Schließlich sei auf das deutsche Geschmacksmuster DE M 93 05 222 hingewiesen, in dem zwei aneinander liegende Pflaster gezeigt sind, die gegebenenfalls über eine Perforationslinie voneinander getrennt sind.

Die zur Zeit im Handel befindlichen Pflasterstrips sind zum größten Teil einzeln eingesiegelt, was mit einem hohen Materialverbrauch und einer relativ aufwendigen Herstellung einhergeht.
Auch haben insbesondere die einzelnen Pflaster den erheblichen Nachteil, daß eine Applizierung mit einer Hand, wie es bei einer Verletzung der anderen zumeist unumgänglich ist, daran scheitert, daß das Siegelpapier nicht zu entfernen ist.

Aufgabe der Erfindung war es daher, Pflaster ohne zusätzliche Einsiegelung durch Siegelpapier zu schaffen, die schnell entnahmebereit, einfach zu handhaben und möglichst durch eine Hand zu applizieren sind.

Gelöst wird diese Aufgabe durch ein Trennpapier mit mehreren auf diesem befindlichen, im wesentlichen parallel nebeneinander angeordneten Pflastern, wie es im Anspruch 1 niedergelegt ist. Die Unteransprüche beschreiben vorteilhafte Ausbildungsformen des Erfindungsgegenstands.

Die Erfindung betrifft demgemäß ein Trennpapier mit mehreren auf diesem befindlichen, im wesentlichen parallel nebeneinander angeordneten Pflastern, die jeweils aus einem Trägermaterial bestehen, das einseitig mit einer selbstklebenden Beschichtung und gegebenenfalls mit einem Wundabdeckmaterial versehen ist.

Das Trennpapier weist zwischen jedem der Pflaster eine Längsperforation auf und es weist eine Querperforation auf, die unterhalb der Pflaster verläuft, wobei die Längsperforation ausgehend von einer Kante des Trennpapiers zumindest bis zur Querperforation ausgebildet ist.

In einer bevorzugten Ausführungsform erstreckt sich die Längsperforation über die gesamte Breite des Trennpapiers.

In einer bevorzugten Ausführungsform verläuft die Querperforation rechtwinklig zu den Längsperforationen und/oder ist mittig unter den Pflastern angeordnet.

In einer alternativen bevorzugten Ausführungsform ist die Querperforation derartig unter den Pflastern angeordnet, daß ein Anfasser vom übrigen Trennpapier abgetrennt wird, der die Abnahme des Pflasters von dem Trennpapier besonders erleichtert.

Die Längsperforationen können einseitig bis zur Querperforation durchgestanzt sein, was die Abtrennung des Pflasters vom verbleibenden Trennpapier vereinfacht.

Das Trennpapier kann weiterhin einseitig bis zur Querperforation in Form der Pflaster gestanzt sein.

Schließlich hat es sich als vorteilhaft erwiesen, wenn die Pflaster durch weitere Längsperforationen miteinander verbunden sind, so daß die Längsperforationen zwischen den Pflastern und die Längsperforationen des Trennpapiers übereinander liegen.
Insbesondere bei der eben geschilderten Ausführungsform kann das Trennpapier beidseitig bis zur Querperforation in Form der Pflaster gestanzt sein.

Schließlich läßt das Trennpapier besonders vorteilhaft in Form einer archimedischen Spirale aufrollen.
Des weiteren können den einzelnen Trennpapiere nebeneinander angeordnet miteinander insbesondere über eine weitere Perforationslinie verbunden sein, so daß sich ein breites Trennpapier ergibt mit mehreren nebeneinander liegenden Pflastern.

Aus der erfindungsgemäßen Anordnung der Pflaster auf dem Trennpapier resultieren eine Vielzahl von Vorteilen.

Durch den Wegfall der einzelnen Einsiegelungen der Pflaster werden erhebliche Materialeinsparungen erreicht.

Dann ist es ohne Schwierigkeiten möglich, ein Pflaster nur mit einer Hand vom Trennpapier aufzunehmen und zu applizieren, ohne daß man mit der Wundauflage in Kontakt kommt.

Als Trennpapiere eignen sich alle bekannten antiadhäsiv ausgerüsteten Siegelpapiere, beispielsweise das Papier BI 60 hg Silox CS 2 DIFFERENTIAL Acrosil.

Anhand der nachfolgend beschriebenen Figuren wird eine besonders vorteilhafte Ausführung des Trennpapiers samt Pflaster näher erläutert, ohne damit die Erfindung unnötig einschränken zu wollen. Es zeigen
- Figur 1: ein längs- und querperforiertes Trennpapier mit insgesamt fünf Pflastern und mittig angeordneter Querperforation,
- Figur 2: ein Trennpapier gemäß Figur 1, wobei zusätzlich eine durchgestanzte Längsperforation vorhanden ist,
- Figur 3: ein längs- und querperforiertes Trennpapier mit insgesamt fünf Pflastern und einer nicht mittig angeordneter Querperforation,
- Figur 4: ein Trennpapier gemäß Figur 3, wobei zusätzlich eine durchgestanzte Längsperforation vorhanden ist,
- Figur 5: ein längs- und querperforiertes Trennpapier mit insgesamt fünf Pflastern und im Kantenbereich der Pflaster angeordneter Querperforation,
- Figur 6: ein Trennpapier gemäß Figur 5, wobei zusätzlich durchgestanzte Längsperforationen vorhanden sind, und zwar zwischen den Pflastern,
- Figur 7: ein Trennpapier, auf dem Pflaster unterschiedlicher Größe angeordnet sind und
- Figur 8: ein Trennpapier, das einseitig bis zur Querperforation in Form der Pflaster gestanzt ist.

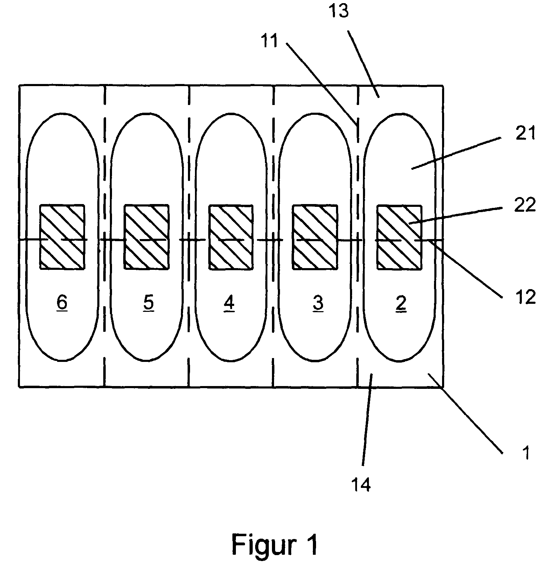

In der Figur 1 ist ein Trennpapier 1 abgebildet, auf dem insgesamt fünf Pflaster 2, 3, 4, 5, 6 parallel nebeneinander angeordnet sind, die jeweils aus einem Trägermaterial 21 bestehen, das einseitig mit einer selbstklebenden Beschichtung und mit einem Wundabdeckmaterial 22 versehen ist.
Das Trennpapier 1 weist zwischen jedem der Pflaster 2, 3, 4, 5, 6 eine durchgehende Längsperforation 11 auf. Weiterhin ist das Trennpapier 1 mit einer Querperforation 12 versehen, die unterhalb der Pflaster 2, 3, 4, 5, 6 verläuft, und zwar in diesem Fall mittig.

Durch die Längsperforationen 11 und die Querperforation 12 können die Pflaster 2, 3, 4, 5, 6 einzeln vom Trennpapier 1 getrennt werden. Dazu wird zunächst am Pflaster 2 das erste Teil 13 des Siegelpapiers durch Trennung der Längsperforation 11 bis zur Querperforation 12 und anschließend Trennung der Querperforation 12 vom verbleibenden Trennpapier 1 abgetrennt.

Der erste Teil 13 des Siegelpapiers dient als Anfasser, mit dem das Pflaster 2 vom zweiten Teil 14 des Siegelpapiers abgezogen wird. Mit der siegelpapierfreien Seite wird das Pflaster 2 derartig auf die Wunde verklebt, daß das Wundabdeckmaterial 22 die Wunde bedeckt und unter gleichzeitigem Abzug des ersten Teils 13 des Siegelpapiers endgültig befestigt.

Der zweite Teil 14 des Siegelpapiers wird durch die vollständige Auftrennung der Längsperforation 11 vom restlichen Trennpapier 1 abgetrennt.

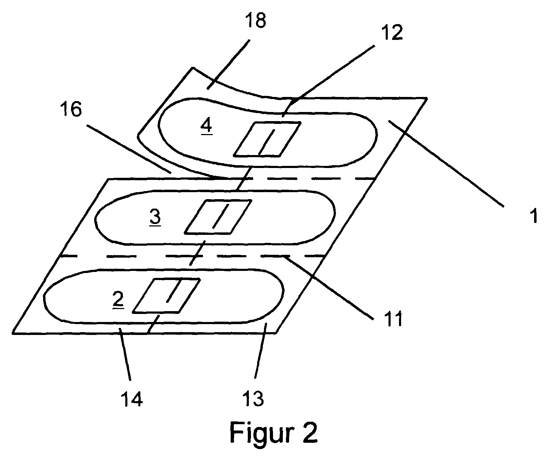

In der Figur 2 ist eine verbesserte Ausführungsformen dargestellt, und zwar ist die Längsperforation 11 zwischen den Pflastern 3 und 4 hälftig bis zur Querperforation 12 durchgestanzt, so daß die Abtrennung des gesamten Pflasters 4 beziehungsweise des Teils 18 des Siegelpapiers besonders einfach erfolgen kann.

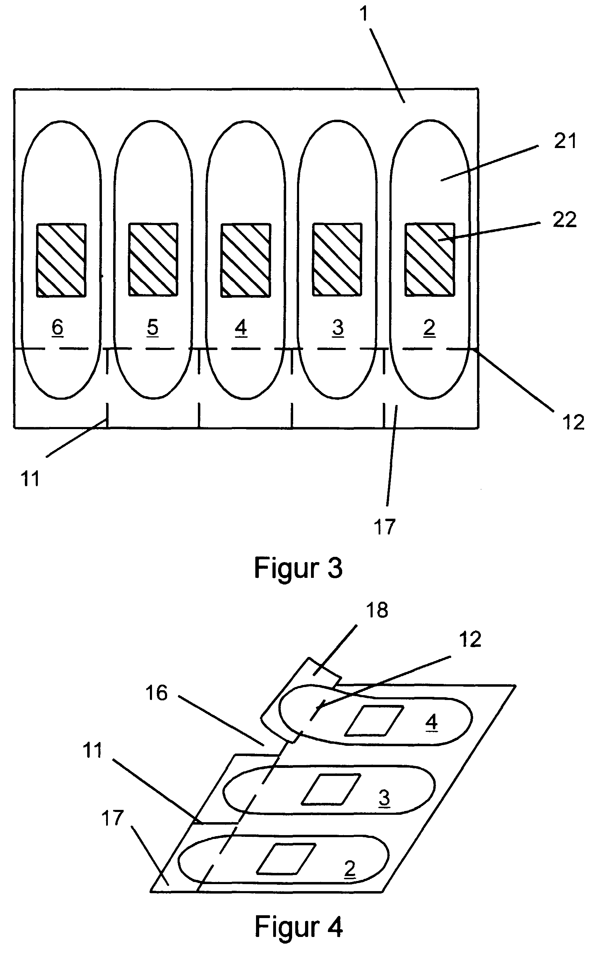

In der Figur 3 ist ein Trennpapier 1 gezeigt, auf dem insgesamt fünf Pflaster 2, 3, 4, 5, 6 parallel nebeneinander angeordnet sind, die jeweils aus einem Trägermaterial 21 bestehen, das einseitig mit einer selbstklebenden Beschichtung und mit einem Wundabdeckmaterial 22 versehen ist.

Das Trennpapier 1 weist zwischen jedem der Pflaster 2, 3, 4, 5, 6 eine durchgehende Längsperforation 11 auf, die ausgehend von einer Kante des Trennpapiers 1 bis zur Querperforation 12 ausgebildet ist, wobei die Querperforation 12 derartig unter den Pflastern 2, 3, 4, 5, 6 angeordnet ist, daß sich Anfasser 17 ergeben, mittels derer die Pflaster 2, 3, 4, 5, 6 besonders vorteilhaft vom Trennpapier 1 abzunehmen sind.

In der Figur 4 ist eine weitere vorteilhafte Ausführungsform gezeigt, in der die Längsperforation 16 durchgestanzt ist. Es ergibt sich ein Anfasser 18, der nur durch Auftrennung der Querperforation 12 abgetrennt wird und zum Abziehen des Pflasters 4 vom Trennpapier 1 dient.

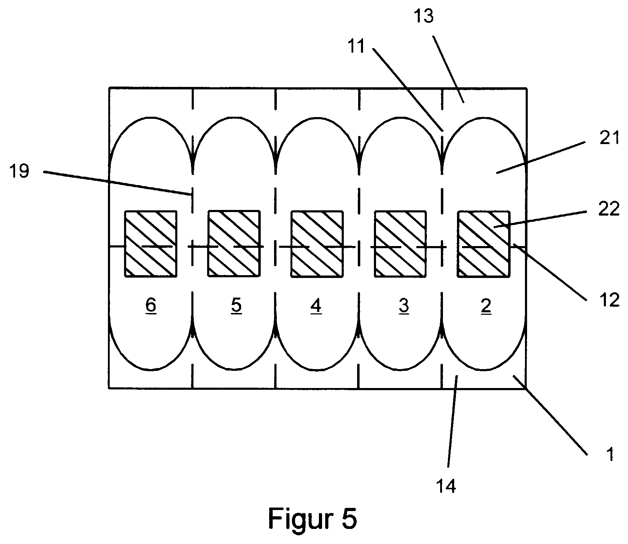

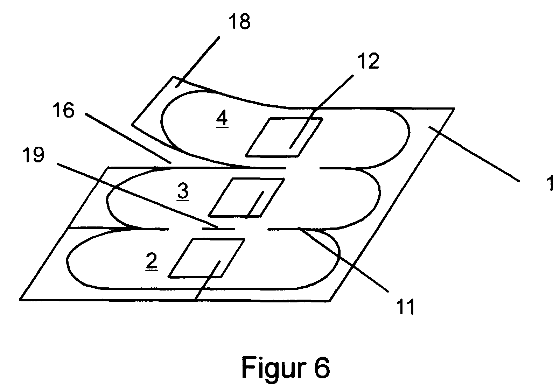

Die Figuren 5 und 6 zeigen die Trennpapiere 1, wie sie bereits in den Figuren 1 und 2 dargestellt worden sind mit dem einzigen Unterschied, daß nunmehr auch die Pflaster 2, 3, 4, 5, 6 mittels weiterer Längsperforationen 19 miteinander verknüpft sind. Die Längsperforationen 19 zwischen den Pflastern 2, 3, 4, 5, 6 und die Längsperforationen 11 des Trennpapiers 1 liegen dabei übereinander, so daß eine Trennung der Pflaster 2, 3, 4, 5, 6 voneinander erfolgen kann, indem beide Perforationen 11, 19 durchtrennt werden.

Bei der Figur 6 ist gemäß Figur 5 auch die Längsperforation 19 zwischen den Pflastern 3 und 4 durchgestanzt, so daß die Abtrennung des gesamten Pflasters 4 beziehungsweise des Teils 18 des Siegelpapiers besonders einfach erfolgen kann.

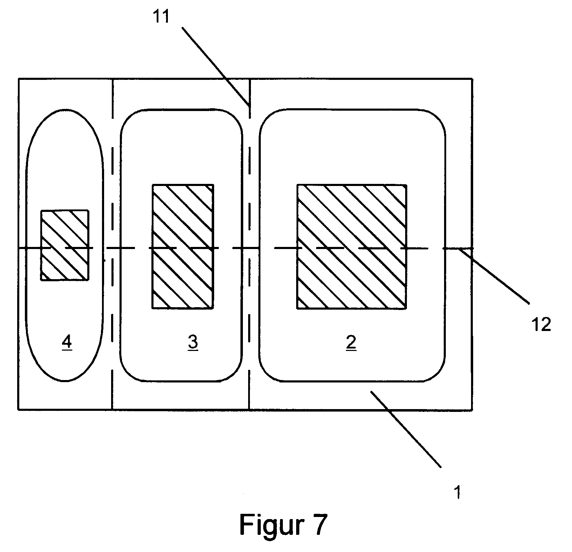

In der Figur 7 ist gezeigt, daß auf dem erfindungsgemäßen Trennpapier 1 ohne Probleme Pflaster 2, 3, 4 unterschiedlicher Größe angeboten werden können.

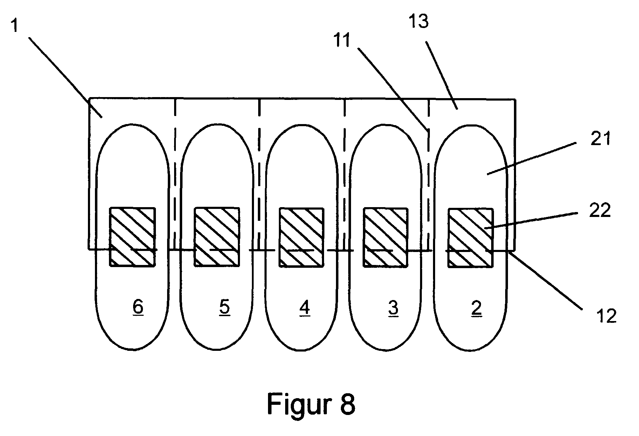

Die Figur 8 stellt ein Trennpapier 1 dar, das einseitig bis zur Querperforation 12 in Form der Pflaster 2, 3, 4, 5, 6 ausgestanzt ist, woraus ein sehr bequem zu händelndes Einhandpflaster entsteht. Durch den Wegfall des überstehenden Randes der zweiten Hälfte 14 des Siegelpapiers entsteht eine gezielte Anfasserhilfe, die optisch klar erkenntlich ist.

Beim Applizieren des Pflasters 2 kann mit einer Hand die gesamte Aufbringung erfolgen. Dabei wird das Pflaster 2 mit dem zweiten Teil 14 von dem sichtbaren Trennpapier 1 abgezogen. Mit der freiwerdenden Klebefläche des abgezogenen Pflasters 2 wird es auf der Haut fixiert, so daß anschließend durch Abziehen des verbleibenden Teils 14 des Siegelpapiers das übrige Pflaster 2 auf die Haut gebracht werden kann.

## Patentansprüche

1. Trennpapier 1 mit mehreren auf dieser befindlichen, im wesentlichen parallel nebeneinander angeordneten Pflastern 2, 3, 4, 5, 6, die jeweils aus einem Trägermaterial 21 bestehen, das einseitig mit einer selbstklebenden Beschichtung und gegebenenfalls mit einem Wundabdeckmaterial 22 versehen ist, wobei das Trennpapier 1 zwischen jedem der Pflaster 2, 3, 4, 5, 6 eine Längsperforation 11 aufweist, wobei das Trennpapier 1 eine Querperforation 12 aufweist, die unterhalb der Pflaster 2, 3, 4, 5, 6 verläuft und wobei die Längsperforation 11 ausgehend von einer Kante des Trennpapiers 1 zumindest bis zur Querperforation 12 ausgebildet ist.

2. Trennpapier nach Anspruch 1, dadurch gekennzeichnet, daß sich die Längsperforation 11 über die gesamte Breite des Trennpapiers 1 erstreckt.

3. Trennpapier nach den Ansprüchen 1 und 2, dadurch gekennzeichnet, daß die Querperforation 12 rechtwinklig zu den Längsperforationen 11 verläuft.

4. Trennpapier nach den Ansprüchen 1 bis 3, dadurch gekennzeichnet, daß die Querperforation 12 mittig unter den Pflastern 2, 3, 4, 5, 6 angeordnet ist.

5. Trennpapier nach den Ansprüchen 1 bis 3, dadurch gekennzeichnet, daß die Querperforation 12 derartig unter den Pflastern 2, 3, 4, 5, 6 angeordnet ist, daß ein Anfasser 17 vom übrigen Trennpapier 1 abgetrennt wird.

6. Trennpapier nach den Ansprüchen 1 bis 5, dadurch gekennzeichnet, daß die Längsperforationen 11 einseitig bis zur Querperforation 12 durchgestanzt sind.

7. Trennpapier nach den Ansprüchen 1 bis 6, dadurch gekennzeichnet, daß das Trennpapier 1 einseitig bis zur Querperforation 12 in Form der Pflaster 2, 3, 4, 5, 6 gestanzt ist.

8. Trennpapier nach den Ansprüchen 1 bis 7, dadurch gekennzeichnet, daß die Pflaster 2, 3, 4, 5, 6 durch weitere Längsperforationen 19 verbunden sind, so daß die Längsperforationen 19 zwischen den Pflastern 2, 3, 4, 5, 6 und die Längsperforationen 11 des Trennpapiers 1 übereinander liegen.

9. Trennpapier nach Anspruch 8, dadurch gekennzeichnet, daß das Trennpapier 1 beidseitig bis zur Querperforation 12 in Form der Pflaster 2, 3, 4, 5, 6 gestanzt ist.
